Europäisches Patentamt

European Patent Office

Office européen des brevets

(11) Veröffentlichungsnummer: **0 478 906 A2**

## (12) EUROPÄISCHE PATENTANMELDUNG

(21) Anmeldenummer: **91111745.5**

(22) Anmeldetag: **15.07.91**

(30) Priorität: **21.08.90 CH 2729/90**

(43) Veröffentlichungstag der Anmeldung:
**08.04.92 Patentblatt 92/15**

(84) Benannte Vertragsstaaten:
**AT BE CH DE DK ES FR GB GR IT LI LU NL SE**

(51) Int. Cl.5: **A61K 7/48, A61K 7/40**

(71) Anmelder: **REALAR ETABLISSEMENT**
**Aeulestrasse 38**
**FL-9490 Vaduz(LI)**

(72) Erfinder: **Süess, Hans R., Dr.**
**Hardstrasse 77**
**CH-4657 Dulliken(CH)**
Erfinder: **Lene, Howard L.**
**Aeulestrasse 38**
**FL-9490 Vaduz(LI)**

(74) Vertreter: **EGLI-EUROPEAN PATENT**
**ATTORNEYS**
**Horneggstrasse 4**
**CH-8008 Zürich(CH)**

(54) **Intra-Sorptionsmittel.**

㊵ Das Intra-Sorptionsmittel oder Trägermaterial dient zur Einbringung oder Fixierung von Substanzen in eine Matrix in einer derartigen Weise, dass eine Abgabe aus demselben in das Substrat nicht stattfindet.

Unter dem neuen Ausdruck Intra-Sorption ist ein Sorptionsvorgang beschrieben, bei dem eine Substanz innerhalb einer Matrix durch einen anderen mit ihm verbundenen Stoff selektiv aufgenommen wird.

Bei einer Verwendung dieses Mittels wird durch eine Substanz eine Hautbräunung vorgetäuscht, die allein mit Seifenwasser abwaschbar ist. Die Bräunung ist aber resistent gegenüber Wasser, Schweiss oder Reibung.

Das Intra-Sorptionsmittel ist insbesondere für Kosmetika, d. h. für die Haut, die Haare, die Nägel usw., jedoch auch z. B. in der Dermatologie zur Behebung von Hautkrankheiten vorgesehen. Es ist aber auch zum Auftragen auf Holzflächen, Leder, Kunststoffe usw. verwendbar und lässt sich auch von diesen Flächen z.B. mittels Seifenwasser abwaschen.

EP 0 478 906 A2

Die Erfindung betriffte ein Intra-Sorptionsmittel gemäss dem Oberbegriff des ersten Patentanspruches.

Ein Intra-Sorptionsmittel oder Trägermaterial, dient zur Einbringung und Fixierung von Substanzen in eine Matrix in einer derartigen Weise, dass eine Abgabe aus demselben in das Substrat nicht stattfindet.

Der Ausdruck Intra-Sorption ist eine neue Wortschöpfung und beschreibt einen Sorptionsvorgang, bei dem eine Substanz innerhalb einer Matrix durch einen anderen mit ihm in Verbindung stehenden Stoff selektiv aufgenommen wird.

Dabei wird ein neuer Weg beschritten, wobei ein bestimmtes Material, z.B. eine Farbe, mit einem Substrat, wie Haut, Nägel, Leder oder Platten aus Holz oder Kunststoff, direkt in innige Verbindung treten kann, und dabei in oder auf dieses Substrat nicht sorbiert wird oder sich durch dieses ausbreitet.

Die Absorption durch die Haut oder andere durchlässige Substrate von Wirkstoffen aus Präparaten, die der Verschönerung, der Pflege oder Behandlung dienen, stellt oft ein Problem dar. So führt die epidermale Absorption zu Hautverfärbungen und eventuell zu toxischen Folgeerscheinungen.

Die Absorption von stark riechenden Stoffen, wie z.B. Merkaptanen, führt zur andauernden Abgabe von übelriechenden Substanzen von der Haut. Die Hautabsorption vieler anderer Substanzen kann zu Verfärbungen, Juckreiz, Brennen, sodann zu allergischen Reaktionen, dann zu mutagenen, teratogenen und carcinogenen Erscheinungen führen.

Bei anderen Substraten, wie Haar oder Nägel, Leder und anderen durchlässigen Materialien kann eine unerwünschte Absorption Schwellungen, Rissbildung, Schälung, Aufspaltung, Fleckenbildung, Trübung, Strukturschäden, Verfärbung und anderes mehr, zur Folge haben.

Bekannte Mittel dieser Art werden beispielsweise kosmetisch zum Auftragen von die Haut färbenden Substanzen verwendet. Diese sogenannten Bräunungsmittel sind grundsätzlich in zwei Gruppen aufzuteilen, von welchen die erste Gruppe aus

a) Mitteln besteht, die durch Wasser leicht zu entfernen sind, während die zweite Gruppe aus

b) Mitteln besteht, die nicht durch Wasser entfernt werden können.

Die zur Gruppe a) gehörenden Mittel haben den Nachteil, dass sie durch leichte Berührung mit Wasser und z.B. beim Schwitzen oder Schwimmen entfernt werden können. Deshalb sind sie für kosmetische Zwecke nur bedingt verwendbar, weil sie z.B. bei einem festlichen Anlass dauernd überwacht und nachgetragen werden müssen.

Zur Kategorie a) gehören z.B. Bräunungskremen, die der Trägerin oder dem Träger vorübergehend eine gebräunte Haut vermittelt.

Zur Gruppe b) gehören Mittel, die beispielsweise in der DE-OS 16 17 431 beschrieben sind. Dieses Produkt ist durch Wasser nicht zu entfernen, und hat besipielsweise den Zweck, die häufig gewaschenen Hände von Zahnärzten zu schützen.

EP-PS 0 197 485 beschreibt ein weiteres Mittel, das gegen Wasserberührung, wie beim Schwitzen, Schwimmen und Waschen, widerstandsfähig ist.

Es ist aber normalerweise wünschenswert, ein auf die Haut aufgetragenes Mittel dann zu entfernen, wenn es nicht mehr benötigt wird. Dies kann z.B. nach einem festlichen Anlass oder einem Ausflug der Fall sein und ist deshalb wichtig, weil eine Beschichtung der Haut für diese auf die Dauer Nebenwirkungen haben kann. Die DE-OS gibt keine Hinweise, wie das Mittel schonungslos entfernt werden kann. Es ist aber zu erwähnen, dass wasserfreie, sogenannte Entfernungsmittel u.U. für die Haut schädlich sein können.

Aufgabe der Erfindung ist somit die Schaffung eines Mittels, das die Nachteile bisheriger Ausführungen nicht aufweist.

Dabei soll das zu schaffende Mittel mit einer Matrix versehen sein, die

- geruchlos, durchsichtig und nicht fettig ist,
- sich nicht an der Hautfläche festsetzt oder sich mit ihr verbindet,
- nicht feststellbar oder fühlbar ist, und nicht durch Reiben entfernbar ist, und
- keinen sichtbaren Rand bildet.

Ferner soll das Mittel auch auf andere Substrate, wie Haare, Nägel, Holz und Kunststoff auftragbar sein.

Diese Aufgabe ist erfindungsgemäss durch die Merkmale im Kennzeichnungsteil des ersten Anspruches gelöst.

Ausführungsformen sind in den abhängigen Ansprüchen umschrieben.

Die geschaffene Ausführung ist somit wasserbeständig, jedoch erfordert sie keine die Haut schädigende Mittel für die Entfernung, weil oberflächenaktive Reinigungsmittel (surfactants) genügen.

Es wurde nun gefunden, dass man z.B. eine Bräunung der Haut vortäuschen kann, indem man auf die Haut oder ein anderes permeables Substrat ein Trägermaterial bringt, in dem die Wirksubstanzen, wie nachfolgend beschrieben, durch Intra-Sorption festgehalten werden.

Die Intra-Sorption kommt dadurch zustande, dass die Wirksubstanz in eine Co-Sorptionssubstanz eingeschlossen auf dem Substrat aufliegt, aber damit nicht direkt in Berührung kommt. Das Trägermaterial andererseits lagert sich an verschiedenen hydrophilen, lipophilen, proteophilen oder anderen Bestandteilen permeabler Substrate, wie Haut, Haare, Nägel, Leder oder gewisse künstliche Materialien, an.

Derart fixierte Wirkstoffe verhalten sich, wenn auf die Haut oder ein anderes Substrat aufgebracht, als ob sie ein Bestandteil desselben wären, auch wenn sie weder direkt an das Substrat gebunden noch von diesem absorbiert werden. Dies ist deshalb so, weil die Wirkstoffe in der Matrix, gebildet durch das Trägermaterial, "gefangen" sind und nur durch die Matrix selbst mit dem Substrat verbunden sind.

Ferner wurde festgestellt, dass durch gewisse Modifikationsmittel, wie z.B. Dispergiermittel, eine mögliche Absorption weiter vermindert werden kann, weil sie offenbar in der Lage sind, die Aktivstoffe in der Matrix festzuhalten.

Während die in die Matrix eingebetteten Wirkstoffe durch Einflussnahmen wie Reiben, Schwitzen oder Wasserberührung, nicht entfernt werden, sind sie durch Einwirkung von oberflächenaktiven Substanzen, wie Seife und Detergentien, leicht entfernbar. In dieser Hinsicht liegt ein von Substantivität verschiedenes Verhalten vor, das darin besteht, dass ein aufgetragenes Material an der Applikationsstelle fest haftet und deshalb kaum mehr entfernt werden kann.

Die notwendigen Eigenschaften und Bestandteile des Intra-Sorptionsmittels beinhalten:

a) Aktivstoffe, welche die gewünschte Wirkung ausüben

b) Nichtflüchtige oder schwerflüchtige Komponenten

c) Modifizierungsmittel

d) Flüchtige Anteile

a) Aktivstoffe sind Wirkstoffe, die in Intra-Sorptionsmittel eingebettet werden können, sie umfassen einen weiten Bereich von in wässrigem und nichtwässrigem Milieu löslichen aber auch nichtlöslichen Substanzen, die kosmetische, reinigende, pflegende , schützende, medizinische oder andere Wirkungen ausüben.

b) Nichtflüchtige oder schwerflüchtige Anteile bilden eine Matrix für die Wirkstoffe, die eine Affinität zum Substrat haben. Die nicht- oder schwerflüchtigen Komponenten, allein oder in Zusammenwirkung mit Modifiziermitteln, schliessen die Wirksubstanzen ein und verhindern die Möglichkeit einer Absorption durch das Substrat, wobei durch die Affinität der nicht oder schwerflüchtigen Anteile zum Substrat ein Eindringen der Wirkstoffe in dasselbe ohnehin schon erschwert ist.

Damit eine erhöhte Verbraucherakzeptanz und Nutzanwendung gewährleistet sind, müssen ferner die nicht- oder schwerflüchtigen Komponenten allein oder zusammen mit dem Modifizierungsmittel als solches in kurzer Zeit nicht mehr oder nur in einem vom Verbraucher akzeptierbaren Ausmass wahrnehmbar sein.

c) Die Modifizierungsmittel können auch nützliche und wertvolle Funktionen ausüben. So wurde z.B. festgestellt, dass Modifizierungsmittel, wie gewisse Detergentien, die Einbettung der "Aktivsubstanzen" in die nichtflüchtige Matrix verstärken können, wodurch die Abgabe und somit die Absorption durch das Substrat zusätzlich vermindert wird.

Ferner können Modifizierungsmittel eingesetzt werden, um die Eigenschaften der Aktivsubstanzen und der nichtflüchtigen oder flüchtigen Zusatzstoffe zu verändern.

d) Die flüchtigen Komponenten werden sowohl aus ästhetischen Gründen benötigt, um die Aufbringung des Intra-Sorptionsmaterials auf das Substrat zu erleichtern, als auch nach deren Abdunstung eine ausreichende Deckkraft und eine befriedigende Haftung des Materials auf dem Substrat zu gewährleisten. Die flüchtige Komponente kann noch weitere nützliche Wirkungen, wie die Vorbereitung der Substratoberfläche zur Aufnahme des Intra-Sorptionsmittels ausüben, sie kann ferner antimikrobielle oder ähnliche Eigenschaften, Wärme- und Kältegefühle auf der Haut bewirken, sowie weitere nützliche Eigenschaften aufweisen.

Beispiele von Bestandteilen

A) Aktivstoffe

Die Aktivstoffe umfassen ein weites Gebiet von wässrigen und nichtwässrigen, löslichen und unlöslichen Bestandteilen, die verschönernde, kosmetische, reinigende, behandelnde, schützende, medizinische oder andere Effekte hervorrufen, wobei sich die Eigenschaft, dass sie vom Substrat, auf das sie aufgetragen werden, kaum absorbiert werden, als Vorteil erweist.

Dazu gehören u.a.:

a1) glanzerzeugende oder glanzvermindernde Stoffe

b1) Oel und Feuchtigkeit absorbierende Mittel

c1) Materialien, um den Verlust von Feuchtigkeiten, Oelen und anderen flüchtigen oder auswaschbaren Substanzen herabzusetzen

d1) Stoffe zur Abstossung, zum Einbetten, Dispergieren oder Zerteilen von Wasser, Wasserdampf, Schmutz, Gasen, Mikroben und Viren oder anderer unerwünschter Materialien.

e1) Für Lebensmittel, Arzneistoffe und Kosmetika zugelassene, sowie industriell benötigte Farbstoffe, Pigmente, Irideszens-Pigmente, Flüssigkristalle, Bio-Farbstoffe (z.B. Melanin), ferner Farbstoffe entwikkelnde oder verstärkende Substanzen.

f1) Wärme, Strahlungen oder andere energieabsorbierende, schützende, zerstreuende oder abstossende Materialien sowie auch antioxydative, antireduzierende, antikorrosive und weitere vor exogenen oder endogenen Angriffen schützende Stoffe.

g1) geschmeidigmachende und straffende Wirkstoffe

h1) Duftstoffe und andere riechende Materialien

i1) Auch Stoffe zur Absorption, Veränderungen oder Wirkungsverlängerung von wohlriechenden oder stinkenden Verbindungen.

j1) Kosmetische, das Wohlbefinden steigernde oder medizinische Wirkstoffe, Insektizide, wie z.B. Peliculocide usw., bei denen eine oder mehrere Wirkungen günstig beeinflusst werden, wenn deren Absorption durch die Haut, Haare, Nägel, Membranen oder andere Substrate verhindert oder vermindert wird oder wenn dadurch ein unerwarteter oder neuer Effekt auftritt.

k1) Polierende, sowie andere, die Oberfläche verbessernde oder wieder herstellende, die Abnützung vermindernde, sowie andere Materialien.

Oft kann es wünschenswert sein, obwohl dies keine notwendige Voraussetzung ist, dass die Aktivsubstanzen eine geringe Löslichkeit in der nichtflüchtigen oder schwerflüchtigen Komponente haben.

B) Nichtflüchtige oder schwerflüchtige Komponenten

Diese bilden eine Matrix für die "aktiven" Bestandteile. Sie sollen eine gewisse Affinität zum Substrat aufweisen. Die nichtflüchtige oder schwerflüchtige Komponente, allein oder im Zusammenwirken mit Modifizierungsmitteln, halten die Aktivstoffe fest und verhindern die Möglichkeit einer Absorption durch das Substrat. Gleichzeitig bewirkt jedoch auch die Affinität der nicht- oder schwerflüchtigen Komponente zum Substrat ein Festhalten der "gefangenen" Wirkstoffe.

Oft mag es wünschenswert sein, obschon dies keine notwendige Voraussetzung ist, dass die Aktivsubstanz eine gewisse Löslichkeit in der nicht- oder schwerflüchtigen Komponente aufweist.

Damit eine verstärkte Akzeptanz durch den Anwender und ein erhöhter Nutzen erzielt werden kann, muss die nicht- oder schwerflüchtige Komponente für sich oder zusammen mit den Modifizierungsmitteln auf dem Substrat ferner nicht mehr feststellbar oder durch den Anwender nicht mehr oder nur noch in einem akzeptierbaren Ausmass wahrnehmbar sein.

Durch eine oder mehrere quantifizierbare Prüfungen muss nachweisbar sein, ob die nicht- oder schwerflüchtigen Komponenten diesen Ansprüchen genügen. So sind Tests entwickelt worden, um die Sichtbarkeit, die Klebrigkeit, die Fettigkeit, die Oberflächenveränderung und andere Eigenschaften festzustellen. Diese Prüfverfahren erleichtern die Auffindung von Materialien, die nach der Auftragung auf ein Substrat in sehr kurzer Zeit praktisch nicht mehr oder nur in einem akzeptablen Masse vom Anwender wahrgenommen werden können.

Beispiele solcher Bestandteile umfassen u.a:

a2) Natürlich vorkommende Lipide menschlicher, tierischer oder maritimer Herkunft, dann auch solche, die von Insekten, Mikroben, Pilzen, Viren oder anderer, sich fortpflanzenden Wesen stammen. Beispiele sind: Ceramide, Sphinogolipide, Wachs- und Sterolester, sodann Lipide, die dem Intercellularraum, den Ganglien und Membranen entnommen wurden. Sodann diesen verwandten Lipide und ihre natürlichen oder durch Bio-Technologie oder Synthese gewonnenen Formen oder Abkömmlinge.

b2) Durch Bio- oder Gentechnologie gewonnene, natürliche Lipide, Wachse und andere halbfeste Verbindungen, die ursprünglich synthetisch hergestellt wurden oder menschlicher, tierischer, maritimer und pflanzlicher Herkunft sind oder aus Insekten, Mikroben, Pilzen, Viren oder anderen sich fortpflanzenden Quellen stammen, sowie deren Derivate.

c2) Lipide synthetischer Herkunft, Wachse, wie Polyethylenwachse oder hydriertes Jojobaöl und andere Materialien synthetischer Herkunft oder solche, die synthetische Varianten oder Abkömmlinge von Stoffen sind, die von Menschen, Tieren, Meeresbewohnern, Pflanzen, Insekten, Mikroben, Pilzen, Viren oder anderen reproduzierbaren Substanzen stammen.

d2) Höhere Petroleumfaktoren, andere Kohlenwasserstoffe und deren Derivate, wie Wachse und auch Vaseline mit einem verringerten Gehalt flüssiger, paraffinölartiger Anteile.

e2) Reine und modifizierte Silicone und solche, an welche vernetzende und funktionelle Gruppen angelagert sind, solange sie die weiter oben genannten Voraussetzungen erfüllen.

C) Flüchtige Bestandteile

Die flüchtigen Bestandteile werden gebraucht, um die Auftragung des Systems auf das Substrat zu erleichtern (z.B. durch Herabsetzung der Viskosität, durch Erhöhung des Spreitvermögens) um sinnliche Eindrücke entstehen zu lassen, wie Gerüche, Kälte, Wärme; dann, damit nach der Abdunstung eine gleichmässige Abdeckung und eine ausreichende Haftung auf dem Substrat erreicht wird.

Die flüchtigen Anteile können andere wünschenswerte Wirkungen haben, wie z.B. die Vorbereitung der Substratoberfläche, um das Intrasorptionsmaterial aufzunehmen. Sie können antimikrobische und verwandte Eigenschaften haben, sowie weitere nützliche Dienste leisten.

Beispiele flüchtiger Anteile umfassen, sind aber nicht beschränkt auf: Halogenkohlenwasserstoffe, Siloxane, wie Cyclomethicone, Derivate flüchtiger Siloxane, Ether, wie Dibuthylether, Alkohole, wie Ethanol und Isopropanol, Ester, wie Ethylacetat, Ketone, wie Methyl-Ethylketon, Wasser und weitere nützliche, flüchtige Verbindungen.

D) Modifizierungsmittel

Die Modifizierungsmittel haben eine Reihe nützlicher und wertvoller Eigenschaften. So können Modifizierungsmittel, wie gewisse Dispergiermittel, die Einschliessung der Aktivsubstanzen verstärken, wie z.B. das schützende, aber die Haut verfärbende Gentianaviolett in der nichtflüchtigen Matrix. Dadurch wird die Einschliessung verstärkt und die Gefahr einer Hautverfärbung und Absorption weiter vermindert. Andere Modifizierungsmittel können zugegeben werden, um folgende Eigenschaften zu verändern. Säuren können angewandt werden, um die Färbung von Farbstoffen zu verändern. Andere Zusätze verändern die Eigenschaften der nichtflüchtigen Anteile, indem z.B. Geliermittel zugegeben werden, um die Spreitung herabzusetzen oder es können Aromatika zu den flüchtigen Anteilen gegeben werden, um den Kühleffekt zu erhöhen oder um andere Eigenschaften zu verstärken, wie z.B. die Zugabe von Konservierungsmitteln.

Beispiele derartiger Modifizierungsmittel sind u.a.:

a3) Dispergiermittel, wie oberflächenaktive Verbindungen mit einem hohen HLB-Wert: Anionische Detergentien (z.B. Alkylsäure, Alkylphosphate). Nichtionogene, wie die Polysorbate und hochethoxylierte Polyoxethylenester und -ether. Amphotere, wie Alkylimidazoline und Acylpeptide und kationische, oberflächenaktive Verbindungen, wie ethoxylierte Alkyldimethylammoniumsalze.

Andererseits benötigen gewisse Systeme Dispergiermittel mit einem niedrigen HLB-Wert, wie Sorbitanfettsäure-Ester und Ether.

b3) Gelierende und die Viskosität erhöhende Zusätze, wie Carbomere, Alginate, synthetische Cellulose-Acrylpolymere und natürliche wasserlösliche Polymere und Geliermittel auf der Basis von Aluminiumsalzen von Fettsäuren.

c3) Puffer, wie Acetate, Citrate, Phosphate, Alpha-Hydroxysäuren usw.

d3) Feuchthaltemittel, Weichmacher und plastifizierende Materialien, wie Polyethylenglycole, Glycerin, Propylen-, Butylen- und Hexylenglycole, Sorbitol, Mannitol und Pentaerythrol oder auch hydrophile oder lipophile Polyoxypropylen-Ether.

e3) Flüchtige Aromatika, wie Menthol, Campher, Menthylsalicylat, Eugenol und ähnliche Verbindungen, auch Riechstoffgemische, Gewürze usw. sowie zugelassene Farbstoffe und Pigmente.

f3) Konservierungsmittel, wie Parabene, Benzoesäure und Sorbinsäure, Aldehyde und aldehydabspaltende Verbindungen, Imidazolidinyl-Harnstoff, Methylchlorisothiazolinone und Methylisothiazolinone oder Antioxydantien, wie Bisulfite, Tocopherole, Propylgallat usw.

Anwendungsbeispiele dieser Erfindung

Typische Anwendungsbeispiele folgen weiter unten.

a4) Beispiel 1 zeigt die Anwendung dieser Technik auf das bekannte antimikrobisch sehr wirksame Gentianaviolett. Es wird heute nur in beschränktem Masse auf der Haut eingesetzt, weil es sie purpurrot verfärbt. Frühere Versuche, diese Nachteile zu beheben, führten zu einer Inaktivierung des Wirkstoffes. Wenn es jedoch, wie in Beispiel 1 gezeigt, im Intrasorptionssystem abgewendet wird, behält es seine biologische Aktivität in der wässrigen Phase; die Intrasorptions-Matrix verhindert jedoch eine Absorption und damit die Verfärbung der Haut.

b4) Eine andere, ähnliche Intrasorptionstechnik zeigt Beispiel 4. Der antimikrobisch wirkende Polyvinylpyrrolidon-Iod-Komplex verfärbt die Haut deutlich weniger stark, wenn er im Intarsorptionssystem vorliegt. Seine antimikrobielle Wirksamkeit ist jedoch weiterhin vorhanden.

Wie schon erwähnt, kann diese Erfindung nicht nur auf der Haut, sondern auch im Haar, auf den Nägeln oder anderen proteinhaltigen Oberflächen zur Anwendung kommen. Aber auch andere Oberflächen, wie die von Holz, von anderen cellulosehaltigen Stoffen, Kunststoffen, Metallen oder Keramik kommen in Frage.

Der Gebrauch von Farbstoffen zur Erzielung einer Farbgebung ohne Verfärbung oder Verflechtung des Substrates ist eine weitere Anwendung. Beispiel 2 zeigt dies für organische Farbstoffe und Beispiel 3 für Pigmente.

In beiden Fällen ist die der Haut verliehene Farbgebung aussergewöhnlich natürlich, weil die organischen Farbstoffe von Beispiel 2 und die anorganischen Pigmente in Beispiel 3 in mikrodisperser Verteilung in der Intrasorptions-Matrix vorliegen, welche selber in ausserordentlich engem Kontakt mit der Haut steht. Der überraschend natürliche Farbeffekt wird noch dadurch erhöht, weil das Intrasorptions-System die Farbstoffe daran hindert, sich mit der Haut zu verbinden oder in diese einzudringen. Auch tritt weder eine Anhäufung, eine Fleckenbildung, ein Aufbrechen einer Schicht noch ein vorzeitiges oder ungleichmässiges Verschwinden auf, was alles zu einem unnatürlichen Erscheinungsbild führen würde.

Beispiele 3 und 5 zeigen den Einsatz des Intrasorptionssystems zur Vermeidung von Strahlenschäden und zwar Beispiel 5 beim Benzophenone-2 UV Absorber und Beispiel 3 beim Eisenoxyd/Titanoxyd Pigment, welche die UV-Strahlung blockieren bzw. reflektieren, zusammen mit dem Antioxydans Tocopherolacetat, das als freie Radikale-Fänger wirkt. Freie Radikale entstehen regelmässig bei biologischen Prozessen; deren Auftreten wird jedoch durch die UV-Strahlung stark erhöht. Das Intrasorptionssystem kann sowohl für UV-Filter, zur Unterdrückung der Bildung freier Radikale sowie zu deren Unschädlichmachung eingesetzt werden. Sodann reduziert die Intrasorptionstechnologie den Verlust an Wirksamkeit, die normalerweise durch die Absorption von Filtersubstanzen durch die Haut hervorgerufen wird. Auch wird die Gleichmässigkeit des Hautschutzes gewährleistet. Dieser Vorteil ist deshalb besonders wichtig, weil die Verminderung des UV-Schutzes durch Absorption auch einen verminderten Schutz des Immunsystems bedeutet. Die Absorption der Filtersubstanzen durch die Haut kann auch zu Hautreizungen und Ekzemen führen und bei Langzeitanwendung sind toxische Erscheinungen nicht auszuschliessen.

Weitere Beispiele der Erfindung

A1) Schutz und Wirkungsverlängerung von Duftstoffen

In das Intrasorptionssystem können auch Duftstoffe eingebracht und dort festgehalten werden, womit eine Bindung an protein- oder lipidhaltige Hautstellen oder an Cellulose, Kunststoffe oder andere Materialien, welche als Substrat wirken, verhindert wird. Eine Bindung führt normalerweise zu einem Verlust an Riechstoffintensität oder es kann dadurch eine unerwünschte Veränderung des Riechstoffcharakters hervorgerufen werden. Dies kann sowohl bei einzelnen Riechstoffkomponenten wie auch bei Riechstoffgemischen, aus welchen die meisten Parfums bestehen, auftreten. Das Endresultat ist ein geschmälertes Geruchserlebnis im Vergleich zu dem, wie es beim Riechen an der Verkaufspackung erlebt wird.

Dies ist in Fig. 6 festgehalten. Der Riechstoff wird vor solchen Einwirkungen geschützt, womit ein ansprechendes Dufterlebnis erzielt wird. Das Intrasorptionssystem kann nun auch in der Weise modifiziert werden, dass die Duftstoffabgabe beschleunigt oder verlangsamt wird. Diese Konzeption kann sowohl bei normalen Duftstoffen, wie auch bei Deodorants und Geruchvernichtern angewandt werden.

B1) Eine andere Anwendung dieser Erfindung ist die Wirkstoffabgabe von Insektenabstossmitteln (repellents), wie N,N-Diethyl-m-Toluamid und die Verhinderung von Wirkstoffverlust durch Absorption und Adsorption. Solche Vorteile können auch erzielt werden bei Mitiziden und Parasitiziden, wie Dichlorvos zum Beispiel (6b).

C1) Neben verschiedenen Farben und Pigmenten kann diese Erfindung auch zur Erhöhung visueller Effekte solcher Substanzen verwendet werden, welche Glanz , Metallglanz oder einen Schimmer bewirken (wie z.B. Metallstearate etc.) oder andere visuelle, strukturelle Effekte erzeugen. Auch können sie damit vor zu intensiver Auftragung und nachfolgendem Verlust geschützt werden (Beispiel 6c).

D1) Schutzwirkung. Das Basissystem, exemplifiziert durch die Vehikel in Beispiel 1-6 (jedoch ohne die Aktivstoffe) kann die Haut vor äusseren Einwirkungen schützen durch die besondere Eigenschaft des Intrasorptionssystems, sich an die Haut eng anzulagern, ohne daran fest gebunden zu sein. Es bildet sich nämlich ein praktisch unsichtbarer, fettfreier, nicht klebriger, farb- und geruchloser Schutzfilm, der den Kontakt mit Schmutz, Fett, verfärbende oder Hautreizungen hervorrufende Substanzen und dgl. verzögert.

Dieser Schutzeffekt kann noch vergrössert werden, wenn spezielle Zusätze in das Basissystem inkorporiert werden. So schützt z.B. Chitosan, wenn es in die wässrige Phase eingearbeitet wird, die Haut und deren Anhängsgebilde vor der entfettenden Wirkung organischer Lösungsmittel (Beispiel 6d).

E1) Die bisherigen Anwendungsbeispiele bezogen sich auf die Haut. Die gleichen nützlichen Effekte lassen sich jedoch auch auf anderen Substraten erzielen.

Haar:

Der Schutz gegen übermässige Absorption in das Haar kann beispielsweise einen optimalen Glanz, Weichheit der Haaroberfläche, verminderte Aufspaltung der Haarspitzen, geringere Abtragung der Cuticula zu Folge haben. Auch wird das Auftragen, die gleichmässige Verteilung und die Brillanz von Haarfarben und Pigmenten verbessert, während das Wegbringen von Haarschuppen erleichtert wird.

Holz:

Es lassen sich damit erzielen: Erhöhte Ablagerung von Pflegemitteln, Schutz- und Verschönerung der Holzstruktur, Erhöhung des Glanzes, Hervorhebung der Maserung sowie Abschirmung vor Umwelteinflüssen. Zusätze können die Schutzwirkung weiter steigern hinsichtlich Strahlung sowie Mikroben- und Parasitenbefall.

Kunststoffe: wie Holz

Metalle: wie Holz; zusätzlicher Schutz bei Verwendung von Mitteln zur Vermeidung von Korrosion, Oxydation, Säureangriff usw.

Eine andere Art von Schutzwirkung durch die Intrasorptions-Technologie kann erzielt werden, wenn die Absorption von Werkstoffen derart verlangsamt wird, dass eine sonst auftretende Beeinträchtigung durch eine zu intensive Einwirkung nicht mehr stattfindet. Zum Beispiel die Verminderung der Absorption von Oxydationsmitteln, wie anorganische und organische Peroxyde, von Aldehyden, wie Glutaraldehyd, von Hautreizungen hervorgerufenen Hautrötungsmitteln (Rubefacientia), wie Histamindihydrochlorid und ggfs. hautreizende, aber sehr nützliche Riechstoffe, wie Zimtalkohol, Sassafras-Essenz, Pinienöl usw..

Beispiele und Feststellungen einer Wirkungsbeeinträchtigung
– – – – – – – – – – – – – – – – – – – – – – – – – – – – – – – – – – – –

Es wurde festgestellt, dass gewisse Wirkstoffe das richtige Funktionieren des Intrasorptionsmittels beeinträchtigen können, wobei es möglich ist, solche Einwirkungen festzustellen. So ist z.B. die Fähigkeit des Intrasorptionsmaterials, ein Wandern der eingeschlossenen Substanzen zu verhindern, stark beeinträchtigt, wenn Dimethicone teilweise oder ganz durch Amodimethicone ersetzt wird, ein Siliconpolymer mit einer endständigen Aminogruppe ($-NH-CH_2-CH_2-NH_2$). Ein Anwendungsbeispiel dafür erhält man, wenn die Formel A (die mit Beispiel 2 identisch ist) der Formel 2 gegenübergestellt wird, die auch die gleiche Zusammensetzung aufweist wie 2, jedoch Trimethylallylamodimeththicone-Polymer enthält, anstelle von Dimethicone-Polymeren.

Von beiden Zubereitungen werden ungefähr 0.25 in die Mitte eines runden Filterapparates von 10 - 15 cm Durchmesser gegeben und etwa während 30 min. einziehen gelassen. Alsdann lässt man diese Filter senkrecht gehalten etwa 1 cm breit in
Wasser eintauchen, das eine Temperatur zwischen 20 und 35°C aufweist. Auf diese Weise diffundiert Wasser durch das ganze Filterpapier.

In diesem Versuch zeigt Formel A, welche Dimethicone-Polymere kein Ausbluten enthalten, während die Farbe der Formel B, die Trimethylsilylamodimethicone enthält, ein starkes Auswaschen zeigt und somit einen Hinweis darauf gibt, dass eine Diffusion auf der Haut und eine mögliche Absorption stattfindet.

Unterschiede lediglich in der Polarität können diese Erscheinung nicht erklären, denn es haben gewisse Wirkstoffe, die in den Beispielen erwähnt sind, ausgesprochene polare Charakter und sind in ähnlicher oder höherer Konzentration enthalten, wie das Amodimethicone-Polymere und trotzdem beeinflussen sie das Rückhaltevermögen im Intrasorptionsmaterial nicht.

Solche und andere Tests, die den Widerstand gegen Diffusion, einer Absorption oder ein Wandern aufzeigen, können herangezogen werden, um Wirkstoffe, welche die gewünschte Rückhaltung in der Intrasorptionsschicht beeinträchtigen würden, auszuschliessen. Andererseits können solche Untersuchungen herangezogen werden, um Substanzen zu finden, die das Rückhaltvermögen optimieren.

Beispiel 1 (CTFA-Nomenklatur)

Herabsetzung der Hautverfärbung bei einer Gentianaviolett-Behandlung.

| Nichtflüchtige Komponente | |
|---|---|
| Jojobaöl | 2,0 Teile |
| Dimethicon, hochmolekular | 1,56 Teile |

## Flüchtige Komponente

Cyclomethicon D$_4$ oder D$_5$ oder Mischung
dieser Oligomeren                                                         70,44 Teile

| Aktivsubstanz | |
|---|---|
| Gentianaviolett | 0,5 Teile |

Modifizierungsmittel

| a) Lösungsmittel für Gentianaviolett | |
|---|---|
| Glycerin | 3,0 Teile |
| Glycereth 26 | 2,0 Teile |
| Wasser | 10,85 Teile |

| b) Matrixverstärker zur weiteren Reduzierung der Farbstoffabgabe | |
|---|---|
| PEG-40 Stearat-Ester | 1,5 Teile |
| PEG-25 Lauryl-Ether | 1,5 Teile |
| Polysorbat 20 | 3,0 Teile |
| Polysorbat 40 | 3,0 Teile |

| c) Viskositätserhöhende und plastifizierende Stoffe | |
|---|---|
| Ceteareth-3 | 0,4 Teile |
| Ceteareth-6 | 0,2 Teile |

| d) Konservierungsmittel | |
|---|---|
| Methylchloroisothiazolinon | 0,05 Teile |

Herstellung

Bei 38° - 42° C wird unter Rühren Gentianaviolett in den Lösungsmitteln aufgelöst. Dann werden die Matrixverstärker, die Viskositäts- und Plastizitätsmodifikatoren sowie das Konservierungsmittel zugegeben. Sodann fügt man die nichtflüchtige Komponente zu. Unter Abschluss, um Verdunstungsverluste zu vermeiden, wird die flüchtige Komponente zugesetzt, wobei unter Abkühlung gerührt wird.

Beispiel 2

Oberflächenfärbung ohne Anfärbung des Substrates

| Nichtflüchtige Komponenten | |
|---|---|
| Squalan | 1,8 Teile |
| Dimethicon, sehr hohe Viskosität | 1,3 Teile |

**Flüchtige Komponenten**

Cyclomethicon $D_4$ oder $D_5$ oder Mischung

davon                                               67,4  Teile

| Wirkstoffe | |
|---|---|
| FD&C rot Nr. 33 | 0,033 Teile |
| FD&C gelb Nr. 5 | 0,064 Teile |
| FD&C blau Nr. 1 | 0,003 Teile |

Modifizierungsmittel

| a) Lösungsmittel für die Farbstoffe | |
|---|---|
| Propylenglycol | 3,0 Teile |
| Wasser | 11,8 Teile |

| b) Matrix-Verstärker | |
|---|---|
| PEG-36 Oleat-Ester | 1,4 Teile |
| PEG-20 Cetyl-Ether | 1,6 Teile |
| Polysorbat 20 | 6,2 Teile |
| Natrium Lauryl-Sulfat | 1,4 Teile |

| c) Viskositätssteigernde und plastifizierende Zusätze | |
|---|---|
| Capryl Pyrrolidon | 0,8 Teile |
| Lauryl Pyrrolidon | 1,0 Teile |

| d) Konservierungsmittel | |
|---|---|
| Imidazolidinyl-Urea | 0,2 Teile |
| Wasser | 2,0 Teile |

Herstellung

Bei 38° - 42° C werden unter Rühren die FD&C Farben im Lösungsmittel gelöst, dann werden die Matrixverstärker, die viskositätssteigernden, die plastifizierenden und konservierenden Zusätze beigegeben. Sodann werden die nichtflüchtigen Komponenten zugefügt und zuletzt, in einem geschlossenen Gefäss, um Abdampfungsverluste zu vermeiden, die flüchtigen Komponenten. Es wird gut gerührt, bis eine homogene Masse entsteht - alsdann wird unter Rühren auf Zimmertemperatur abgekühlt.

Beispiel 3

Vertiefung der Gesichtsfarbe und Schutz der Haut durch Pigmente, UV-Dispersionsmittel und Freie-Radikale-Fänger.

| Nichtflüchtige Komponente | |
|---|---|
| PPG 40 Butyl-Ether | 1,9 Teile |
| Dimethyl-Trimethyl-Polysiloxan | 12,0 Teile |

| Flüchtige Komponenten | |
|---|---|
| Cyclomethicon D | 56,75 Teile |

| Aktivstoffe | |
|---|---|
| Wackherr W 9814 Schwarzeisenoxid-Pigment | 0,5 Teile |
| Wackherr W 3801 Roteisenoxid-Pigment | 0,5 Teile |
| Wackherr W 1803 Gelbeisenoxid-Pigment | 0,5 Teile |
| Titandioxid | 1,0 Teile |
| Tocopherol-Acetat | 0,5 Teile |

Modifizierungsmittel

| a) Pigment-Dispergiermittel | |
|---|---|
| Sorbitol | 2,5 Teile |
| Glycereth-26 | 2,0 Teile |
| Wasser | 10,5 Teile |

10

| b) Matrix-Verstärker | |
|---|---|
| PEG-40 Stearat-Ester | 1,5 Teile |
| PEG-25 Lauryl-Ether | 1,5 Teile |
| Polysorbat 20 | 4,5 Teile |
| Polysorbat 40 | 1,5 Teile |
| Natrium-Laurylsulfat | 1,3 Teile |

| c) Konservierungsmittel | |
|---|---|
| Methylchloroisothiazolinon | 0,05 Teile |
| Wasser | 1,0 Teile |

Herstellung

Unter Rühren werden in einem geschlossenen Behälter bei 40 - 45 C die Eisenoxid- und Titandioxid-Pigmente im Dispergiermittel fein verteilt. Alsdann wird der Matrix-Verstärker beigefügt, darauf das im Wasser gelöste Konservierungsmittel zugefügt. Alsdann wird das Tocopherolacetat zu der Mischung der nichtflüchtigen Komponenten gegeben und zugemischt. Zuletzt wird das Cyclomethicon langsam zugefügt, gerührt, bis eine gleichförmige Mischung entstanden ist und dann gekühlt.

Beispiel 4

Nichtabfärbendes Jod/Polyvinylpyrrolidon Desinfektionsmittel

| Nichtflüchtige Komponenten | |
|---|---|
| PPG-40 Butyl-Ether | 1,9 Teile |
| Dimethyl/Trimethyl-Polysiloxan | 12,0 Teile |

| Flüchtige Anteile | |
|---|---|
| Cyclomethicon $D_4$ / $D_5$ | 56,3 Teile |

| Wirkstoffe | |
|---|---|
| Povidone-Jod | 5,0 Teile |

Modifizierungsmittel

Modifizierungsmittel

| a) Povidone-Jod-Lösungsmittel | |
|---|---|
| Glycerin | 2,5 Teile |
| Wasser | 12,5 Teile |

| b) Matrixverstärker | |
|---|---|
| PEG-40 Stearat-Ester | 1,5 Teile |
| PEG-25 Lauryl-Ester | 1,5 Teile |
| Polysorbat 20 | 5,5 Teile |
| Natrium-Laurit-Sulfat | 1,3 Teile |

Herstellung

In einem geschlossenen Behälter werden unter Rühren das Jod/Polyvinyl-Pyrrolidon in der Glycerin-Wassermischung gelöst. Dann werden die Matrixverstärker sowie die nichtflüchtigen Komponenten zugegeben, sodann das Cyclomethicon. Es wird bis zur Gleichmässigkeit gerührt und anschliessend abgekühlt.

Beispiel 5

Wirkungsverlängerung von UV-Filtersubstanzen.
Diese Konzeption verzögert die Wirkungsverminderung, die sich normalerweise infolge der Absorption der UV-Filtersubstanzen durch die Haut ergibt. Dies kann besonders wichtig sein:
a) zum Schutz des Immunsystems der Haut, welcher durch die Absorption der Filtersubstanzen vermindert wird
b) weil die Absorption der Filtersubstanzen zu toxikologischen Problemen führen.

| Nichtflüchtige Komponenten | |
|---|---|
| Dimethicon von ultrahoher Viskosität | 1,56 Teile |

Flüchtige Anteile

Cyclomethicon $D_4$ oder $D_5$ oder deren Mischung                    70,44 Teile

| Wirkstoff | |
|---|---|
| Benzophenon-2 | 1,0 Teile |

Modifikationsmittel

| a) Lösungsmittel für Benzophenon-2 | |
|---|---|
| Glycerin | 3,0 Teile |
| Wasser | 12,85 Teile |

| b) Matrixverstärker zur Verminderung der Absorption | |
|---|---|
| PEG-40 Stearat-Ester | 1,5 Teile |
| PEG-25 Lauryl-Ether | 1,5 Teile |
| Polysorbat 20 | 3,0 Teile |
| Polysorbat 40 | 3,0 Teile |

| c) Viskositätserhöhende und plastifizierende Zusätze | |
|---|---|
| Ceteareth-3 | 0,4 Teile |
| Ceteareth-6 | 0,2 Teile |

| d) Konservierungsmittel | |
|---|---|
| Methylchloroisothiazolinon | 0,05 Teile |
| Wasser | 1,5 Teile |

Herstellung

In einem geschlossenen Behälter wird bei 38° - 42° C das Benzophenon-2 in seinem Lösungsmittel aufgelöst, dann werden die Matrixverstärker, die viskositätserhöhenden und plastifizierenden Komponenten sowie das Konservierungsmittel zugegeben, alsdann die nichtflüchtigen Dimethicon und zuletzt die flüchtigen Anteile. Es wird gerührt, bis eine gleichmässige Masse entsteht und alsdann unter Rühren gekühlt.

Beispiele 6A - 6D

```
Nichtflüchtige Komponenten
  Jojobaöl                              2,0  Teile
  Polysiloxan-Polyether-Copolymer      2,0  Teile
  (Dow Corning Q2-1401)
```

Modifikationsmittel

| a) Lösungsmittel für Gentianaviolett | |
|---|---|
| PEG 400 | 3,0 Teile |
| Glycereth 26 | 2,0 Teile |
| Wasser | 10,0 Teile |

| b) Matrixverstärker | |
|---|---|
| PEG-40 Stearat-Ester | 1,5 Teile |
| PEG-25 Lauryl-Ether | 1,5 Teile |
| Polysorbat 20 | 3,0 Teile |
| Polysorbat 40 | 3,0 Teile |

| c) Viskositätserhöhende und plastifizierende Zusätze | |
|---|---|
| Ceteareth-3 | 0,4 Teile |
| Ceteareth-6 | 0,2 Teile |

| d) Konservierungsmittel | |
|---|---|
| Methylchloroisothiazolinon | 0,05 Teile |

```
Aktivstoffe
(für Beispiel 6A) Riechstoff Nr. 8881156
Luzi AG                                        2,0   Teile
(für Beispiel 6B) N,N-Diethyl-m-Toluamid       4,0   Teile
(für Beispiel 6C) Metallstearat                1,0   Teile
(für Beispiel 6D) Chitosan-Acetat              0,5   Teile
```

| Flüchtige Komponenten | |
|---|---|
| Cyclomethicon (Tetramer) bis total | 100,0 Teile |

Herstellung

In einem geschlossenen Behälter werden unter Rühren die für die entsprechenden Beispiele vorgesehenen Aktivstoffe in den Modifikationsmitteln a), b), c) und d) aufgelöst, dann die nichtflüchtigen Anteile zugegeben und zuletzt das Cyclomethicon (Tetramer). Es wird gerührt, bis eine homogene Masse entstanden ist und hernach abgekühlt.

**Patentansprüche**

1. Intra-Sorptionsmittel an einer Substratfläche, mit einer Matrix mit nicht- oder schwerflüchtigen Komponenten, Modifizierungsmitteln, flüchtigen Anteilen, sowie Aktivstoffen, dadurch gekennzeichnet, dass die Aktivstoffe in der Matrix eingebettet sind, die Matrix an der Substratfläche haftet, jedoch ein Festsetzen oder ein Eindringen der Aktivstoffe in die Substratfläche verhindert, dass die Matrix durchsichtig, visuell jedoch nicht feststellbar oder durch Hautberührung nicht fühlbar ist und dass die Matrix nicht durch Reiben allein, sondern nur mittels oberflächenaktiver Reinigungsmittel entfernbar ist.

2. Sorptionsmittel nach Anspruch 1, dadurch gekennzeichnet, dass es als nicht- oder schwerflüchtige Komponenten mindestens eine Verbindung der folgenden Gruppen enthält:
   a) Lipide,
   b) schwere Petroleumanteile,
   c) Silicone und modifizierte Silicone,

3. Sorptionsmittel nach Anspruch 1, dadurch gekennzeichnet, dass die Modifizierungsmittel aus mindestens einer Verbindung der folgenden Gruppe bestehen:
   a) Dispergiermittel
   b) Gelbildner und die Viskosität erhöhende Mittel,
   c) saure oder basische Puffer,
   d) Anfeuchter und/oder Weichmacher,

14

e) verdampfbare Aromatika und Färbemittel, und

f) Konservierungsmittel, Antioxidationsmittel und Chelate.

4. Sorptionsmittel nach Anspruch 1, dadurch gekennzeichnet, dass als flüchtige Anteile Halogenkohlenwasserstoffe und/oder Siloxane enthalten sind.

5. Sorptionsmittel nach Anspruch 1, dadurch gekennzeichnet, dass als Aktivstoffe wässrige und nichtwässrige, lösliche und unlösliche Zutaten für kosmetische Zwecke enthalten sind.

6. Sorptionsmittel nach Anspruch 2, dadurch gekennzeichnet, dass die Lipide Wachse, Polyethylen-Wachse oder Derivate von natürlichen Materialien, wie hydrierte Jojobaöle und Wachse aus beliebigen Quellen, einschliessen, die beispielsweise humaner, animaler, insektoider, mikrobialer und fungialer Art sein können.

7. Sorptionsmittel nach Anspruch 2, dadurch gekennzeichnet, dass es als schwere Petroleumanteile Wachs, weisse oder gefärbte Vaseline oder auch Vaselinen mit reduzierten Mengen von flüssigen, weissen Ölfraktionen aufweist.

8. Sorptionsmittel nach Anspruch 2, dadurch gekennzeichnet, dass die modifizierten Silicone auch Träger-, Vernetzungs- oder Funktionsgruppen enthältt.

9. Sorptionsmittel nach Anspruch 2, dadurch gekennzeichnet, dass die natürlichen Lipide solche aus unterschiedlichen tierischen oder pflanzlichen Quellen, wie Jojoba, Ceramide, Sphingolipide, Wachs und Sterolester, intracellulare, ganglioside Membrane und verwandte Lipide umfassen.

10. Sorptionsmittel nach Anspruch 2, dadurch gekennzeichnet, dass die Lipide Wachse und andere halbfeste Stoffe umfassen.

11. Sorptionsmittel nach Anspruch 3, dadurch gekennzeichnet, dass das Dispergiermittel eine oberflächenaktive Verbindung mit hohem HLB-Wert, anionischer, nichtionischer, amphoterischer oder kationischer Art ist.

12. Sorptionsmittel nach Anspruch 3, dadurch gekennzeichnet, dass das Dispergiermittel eine oberflächenaktive Verbindung mit niederem HLB-Wert, wie Sorbitanfettsäureester, Glyceride langkettiger Fettsäuren und niedrigethoxylierte Fettsäuren oder Fettalkohole ist.

13. Sorptionsmittel nach Anspruch 3, dadurch gekennzeichnet, dass der Gelbildner, d.h. das die Viskosität erhöhende Mittel, aus Carbomeren oder anderen Acrylpolymerisaten, modifizierter Cellulose, Alginaten sowie anderen Pflanzenschleimen und fettsauren Aluminiumsalze besteht.

14. Sorptionsmittel nach Anspruch 3, dadurch gekennzeichnet, dass die sauren und basischen Puffer Acetate, Citrate, Phosphate oder Alkanolamine aufweisen.

15. Sorptionsmittel nach Anspruch 3, dadurch gekennzeichnet, dass es als Anfeuchter und Weichmacher mindestens eine Verbindung aus den Gruppen der Glykole, Zucker, hydrophilen oder lipophilen Ester und Äther enthält.

16. Sorptionsmittel nach Anspruch 3, dadurch gekennzeichnet, dass es flüchtige Aromatika, wie Menthol, Campher, Methylsalicylate, Eugenol, sowie fertige Düfte, Kräuter und Färbemittel, wie zulässige Farbstoffe und Pigmente enthält.

17. Sorptionsmittel nach Anspruch 2, dadurch gekennzeichnet, dass es flüchtige Halogenkohlenwasserstoffe und Siloxane, Ether, wie Dibutyl-Ether, Alkohole, wie Ethanol und Isopropanol, Ester, wie Ethylacetat, Ketone, wie Aceton, und flüchtige Bestandteile aufweist.

18. Sorptionsmittel nach Anspruch 5, dadurch gekennzeichnet, dass es Substanzen aufweist, die eine Bräunung des Trägers vortäuschen.

**19.** Sorptionsmittel nach Anspruch 5, dadurch gekennzeichnet, dass es Substanzen enthält, die eine medizinische Wirkung auf den Träger ausüben.